Europäisches Patentamt

European Patent Office

Office européen des brevets

(19)

(11) Veröffentlichungsnummer: **0 150 426**
**B1**

(12) **EUROPÄISCHE PATENTSCHRIFT**

(45) Veröffentlichungstag der Patentschrift: **31.10.90**

(21) Anmeldenummer: **84115661.5**

(22) Anmeldetag: **18.12.84**

(51) Int. Cl.⁵: **A 61 L 15/20, A 61 L 15/30, A 61 L 15/44, A 61 K 9/70**

(54) **Wirkstoffabgabesysteme.**

(30) Priorität: **28.12.83 DE 3347277**

(43) Veröffentlichungstag der Anmeldung:
**07.08.85 Patentblatt 85/32**

(45) Bekanntmachung des Hinweises auf die
Patenterteilung:
**31.10.90 Patentblatt 90/44**

(84) Benannte Vertragsstaaten:
**AT BE CH DE FR GB IT LI NL SE**

(56) Entgegenhaltungen:
**EP-A-01 483 91          FR-A-2 497 457
DE-A-2 604 718          GB-A-2 021 950
DE-A-3 007 368          GB-A-2 073 588
DE-A-3 119 752          GB-A-2 095 108
DE-A-3 319 469          US-A-3 742 951
DE-A-31 117 34**

**PATENTS ABSTRACTS OF JAPAN, Band 7, Nr.
155 (C-175)1300r, 7. Juli 1983; & JP - A - 58 67
617 (NITTO DENKI KOGYO K.K.) 22-04-1983**

(73) Patentinhaber: **BAYER AG
D-5090 Leverkusen 1 Bayerwerk (DE)**

(72) Erfinder: **Von Bittera, Miklos
Max-Scheler-Strasse 7
D-5090 Leverkusen 3 (DE)**
Erfinder: **Meyer, Rolf-Volker, Dr.
Buchheimer Strasse 23
D-4150 Krefeld (DE)**
Erfinder: **Dhein, Rolf, Dr.
Deswatinesstrasse 30
D-4150 Krefeld (DE)**

Courier Press, Leamington Spa, England.

**Beschreibung**

Die Erfindung betrifft ein untenstehend näher definiertes medizinisches Pflaster zur transdermalen Verabreichung von Etofenamat.

In der US—A—4 031 894 werden medizinische Pflaster beschrieben, die ein Reservoir aus einer Mischung von Polyisobutenen mit sehr verschiedenen Molgewichten, nämlich M.G. 35 000 bis 50 000 und 1 000 000 bis 1 500 000 und Mineralölen besitzen.

Diese Pflaster sind nur für Wirkstoffe, die in sehr geringen Dosen appliziert werden, geeignet. In der US—PS wird Scopolamin genannt.

In der EP—A—148 391 werden etofenamathaltige Pflaster auf Basis von Polyisobutylen-Polymeren mit bestimmten Molekulargewichtsverteilungen beschrieben.

In der DE—A—3 119 752 werden allgemein Polymere für Wirkstoffabgabesysteme beschrieben. Als Wirkstoff wird Clonidin, ein Mittel zum Senken des Blutdrucks genannt, das in einer bestimmten Teilchengröße im Pflasterpolymeren dispergiert ist. Etofenamat ist ein öliges Antiphlogistikum.

Die Offenlegungsschriften GB—A—2 073 588 bzw. DE—A—3 111 734 betreffen eine Haftklebeschicht von Pflastern zur Applikation von Nitroglyzerin. Als Polymerbasis werden Naturkautschuk oder Naturkautschukverschnitte eingesetzt. Eine Beimischung z.B. von niedrigmolekularen Polyisobutylen (20%) dient hier zur Verbesserung der Kautschukeigenschaften bezüglich Flexibilität und Gasundurchlässigkeit. Naturkautschuke dieser Art sind für Etofenamat nicht geeignet.

In der DE—A—30 07 368 werden Pflaste auf Basis von Styrol/Isopren/Styrol-Blockcopolymeren zur Applikation von Antiphlogistika beschrieben. Als Beispiel wird u.a. Etofenamat genannt. Die Freisetzungsrate dieser Pflaster ist jedoch unbefriedigend.

In der Anmeldung DE—A—26 04 718 werden Wirkstoffabgabesysteme auf Polymerbasis beschrieben, bei denen die Wirkstofffreisetzung über eine mikroporöse Membran gesteuert wird. Für Etofenamat sind solche Systeme ungeeignet.

Die FR—A—2 497 457 offenbart Pflaster zur Applikation von Nitraten (ISDN, PETN), die bei einer befriedigenden Freisetzungsrate für diese Nitrate einen guten Tragkomfort gewährleisten. Dies wird erreicht durch Verwendung von Polymeren, die eine bestimmte Gasübergangstemperatur haben. Diese Bedingung wird zwar von Acrylpolymeren, Isopren-Kautschuken sowie von PIB-Gemischen mit niederem und hohem Molekulargewicht erfüllt, die Freisetzungsrate solcher Polymeren sind jedoch für Etofenamat sehr unbefriedigend.

In der GB—A—2 095 108 werden medizinische Pflaster zur Applikation von Arzneimitteln offenbart. Unter anderem werden in einer allgemeinen Aufzählung auch Antiphlogistika, z.B. Indometacin, genannt. Als Polymerbasis werden allgemein Kautschuke mit bestimmten Glasübergangstemperaturen eingesetzt, die einen guten Tragkomfort gewährleisten, die jedoch bezüglich der Freisetzungsrate von Etofenamat zu wünschen übrig lassen.

Bekannte Wirkstoffabgabesysteme, wie z.B. Gale, Salben, bekannte Pflaster u.ä. erlauben nur eine begrenzte Wirkstoffresorption durch die Haut. Die Resorption hängt von der Grundlage und den Wirkstoffeigenschaften ab.

Es ist eine Aufgabe der vorliegenden Erfindung, medizinische Pflaste zu entwickeln, mit deren Hilfe über einen längeren Zeitraum geregelte, größere therapeutisch wirksame Mengen des Wirkstoffs Etofenamat über die Haut verabreicht werden können. Diese Pflaster sollen mit der Haut verträglich sein und mit ihrer Hilfe soll es möglich sein, hohe therapeutisch wirksame Dosen des Wirkstoffs Etofenamat zu verabreichen.

Überraschenderweise wurde nun gefunden, daß man entsprechende medizinische Pflaster mit deutlich erhöhten Abgaberaten von Etofenamat erhält, wenn man als Polymerkomponente EPDM-Kautschuke einsetzt.

Die Erfindung betrifft ein medizinisches Pflaster zur transdermalen Verabreichung von Antiphlogistika, bestehend aus einer Deckschicht, einer abziehbaren Schutzschicht und einer den Wirkstoff, Schleppmittel und Harze enthaltenden einlagigen Reservoirschicht auf Basis von Polymeren, dadurch gekennzeichnet, daß die Reservoirschicht als Polymere EPDM-Kautschuke enthält und 1 bis 30 Gew.-% des Wirkstoffs Etofenamat enthält.

In Kenntnis des Standes der Technik war es nicht naheliegend, daß die erfindungsgemäß zu verwendenden EPDM-Polymere sich überraschend gut zur transdermalen Applikation von Etofenamat eignen.

Das Wirkstoffreservoir enthält bevorzugt 2 bis 15 Gew.-% Etofenamat, 30 bis 60 Gew.-% Polymerkomponente, 30 bis 60 Gew.-% Schleppmittel und 2 bis 40 Gew.-% Harze, wobei diese Komponenten sich zu 100% ergänzen.

Die erfindungsgemäße einzusetzenden EPDM-Polymeren sind im Prinzip bekannte Produkte, die von verschiedenen Firmen kommerziell angeboten werden.

EPDM-Polymere sind Copolymerisate des Ethylens und/oder Propylens mit weiteren $C_4$-$C_{19}$-α-Olefinen.

Bevorzugt sind EPDM-Kautschuke, die aus 20 bis 90 Gew.-Teilen Ethylen, 10 bis 80 Gew.-Teilen Propylen und 2 bis 15 Gew.-Teilen (besonders bevorzugt 4 bis 10 Gew.-Teile) eines nichtkonjugierten Diens bestehen. Als Dienkomponente in den EPDM-Kautschuken sind aus der Vielzahl möglicher Diene Dicyclopentadien, Ethylidennorbornen und Hexadien-1,4 besonders bevorzugt.

2

Besonders bevorzugt im Sinne der Erfindung einzusetzende EPDM-Polymere sind jene mit Molekulargewichten $M_w$ 20 000 g/mol bis ca. $M_w$ $1 \cdot 10^6$ g/mol, vorzugsweise $M_w$ bis 500 000 g/mol.

Unter Schleppmittel im Sinne der vorliegenden Erfindung werden Öle, Fettsäureester, Triglyceride, Alkohole und oder Fettsäuren verstanden.

Unter Ölen im Sinne der vorliegenden Erfindung werden hochsiedende, aliphatische, araliphatische und/oder aromatische Kohlenwasserstoffe verstanden, vorzugsweise Paraffinöl, Purcellinöl, Perhydrosqualen und Lösungen von mikrokristallinen Wachsen in den Ölen, Mineralöle, bevorzugt Öle, deren Siedebereich zwischen 150°C und 400°C liegt; ferner ungesättigte Kohlenwasserstoffe mit mindestens 16 C-Atomen wie z.B. Oligomere von Monoolefinen wie Tetraisobutylen, Pentaisobutylen, Hexaisobutylen oder auch flüssige Polymerisate aus Dien(Monoen)-(Co)-Polymerisaten. Beispiele für flüssige Polymerisate aus konjugierten Dienen sind solche aus Butadien, Isopren, 1,3-Pentadien, 2,3-Dimethylbutadien, Copolymerisate verschiedener Diene sowie auch flüssige Copolymerisate aus einem konjugierten Diolefin und geringen Mengen von Monoolefinen wie z.B. Buten-1, Isobuten, Hexen-1, Octen-1, Styrol mit MG von 400 bis 6000, vorzugsweise 800 bis 3000 sowie Iodzahlen von 200 bis 500 und Viskositäten von 100—10 000 cP bei 50°C.

Besonders bevorzugt sind flüssige Polybutadien-Polymerisate, die zu mindestens 90% 1,4-verknüpft sind, deren Anteil an cis-Doppelbindungen mehr als 60% beträgt und deren Molmassen 1000—4000 betragen.

Unter Ölen werden auch Silikonöle verschiedener Viskosität, vorzugsweise mit mittleren Molgewichten von 312 bis 15 000, besonders bevorzugt Polydimethylsiloxane, verstanden.

Unter Fettsäureestern werden solche verstanden, die mindestens 12 C-Atome, vorzugsweise 15 bis 46 C-Atome, besonders bevorzugt 16 bis 36 C-Atome enthalten. Insbesondere werden darunter verstanden: Ethylstearat, Laurinsäurehexylester, Dipropylen-glykolpelargonat, Palmitinsäurecetylester, Isopropylmyristat, Isopropylpalmitat, Capryl/Caprinsäureester von gesättigten Fettalkoholen der Kettenlänge $C_{12}$—$C_{18}$, Isopropylstearat, Ölsäureoleylester, Ölsäuredecylester, Ethyloleat, künstliches Entenbürzeldrüsenfett, und zwar jeweils einzeln oder im Gemisch.

Unter Triglyceriden werden reine oder gemischte Ester des Glycerins mit Fettsäuren der Kettenlänge $C_8$—$C_{18}$ verstanden, vorzugsweise Capryl- und/oder Caprinsäuretriglyceride.

Unter Fettsäuren werden gesättigte oder ungesättigte Fettsäuren, vorzugsweise solche mit 12—24 C-Atomen, einzeln oder im Gemisch miteinander, besonders bevorzugt Ölsäure, verstanden.

Unter Ölen im Sinne der Erfindung werden ferner verstanden:

Süßmandelöl, Avocadoöl, Sesamöl, Rizinusöl, Olivenöl, Traubenkernöl, Nelkenöl, Erdnußöl, Maisöl, Haselnußöl, Jojobaöl, Carthamaöl und Weizenkeimöl, jeweils einzeln oder im Gemisch.

Unter Harzen im Sinne der vorliegenden Erfindung werden Kolophonium, dehydriertes Kolophonium, Glycerinester von dehydriertem Kolophonium, Glycerinester von Kolophoniumgummi, hydriertes Kolophonium, Glycerinester von hydriertem Kolophonium, Pentaerythritester von hydriertem Kolophonium, Methylester von hydriertem Kolophonium, polymerisiertes Kolophonium, Glycerinester von polymerisiertem Kolophonium, Terpenharze, Cumaron/Inden-Harze, hydrierte Petroleumharze, mit Maleinsäureanhydrid modifiziertes Kolophonium und Kolophoniumderivate, $C_5$-Petroleumharze und Halbester von Styrol/Maleinsäure-Co-polymeren einzeln oder im Gemisch miteinander verstanden. Besonders bevorzugt werden Polyterpenharze aus Alpha- bzw. Beta-Pinen oder modifizierte Glycerinester der Kolophoniums. Diese Harze können je nach den erforderlichen Eigenschaften hinsichtlich der Klebrigkeit und der Haftfestigkeit auf dem Teil, an dem das resultierende Pflaster angebracht werden soll, entweder allein oder in Kombination miteinander verwendet werden.

Der Wirkstoff Etofenamat kann in einer Menge von 1 bis 30 Gew.-%, vorzugsweise 2—20 Gew.-% in die Reservoirschicht eingearbeitet werden. Die angegebenen Gew.-% beziehen sich auf Gesamtreservoir.

Dem Wirkstoff Etofenamat können zusätzlich noch weitere Wirksubstanzen zugesetzt werden oder auch kühlende oder duftabgebende Substanzen, vorzugsweise Methylsalicylat, Glycolsalicylat, Salicylsäure, Menthol, Pfefferminzöl, Kampfer, Thymol, Acrinol, Scopolaextrakt, Chlorpeniraminmaleat, Benzylnicotinat, Capsiumextrakt, Nonylvanillylamid, Capasaicin.

Erforderlichenfalls können die erfindungsgemäß Pflaster mit Additiven und Füllstoffen, z.B. Alterungsschutzmitteln, Antioxidantien und Verstärkungsfüllstoffen versetzt werden, soweit die gelartigen Eigenschaften nicht zerstört werden.

Bekannte Wirkstoffabgabesysteme, wie z.B. Gele, Salbengrundlagen, Pflaster geben ca. 0,5—5 mg Wirkstoff in 4 Stunden frei. Das oben beschriebene erfindungsgemäße therapeutische System dagegen setzt in 4 Stunden bis zu 18 mg Wirkstoff frei mit einer signifikant größeren Bioverfügbarkeit. Die erfindungsgemäßen Systeme können durch Änderung des Polymeranteils, des Schleppmittels bzw. des Harzes bezüglich ihrer Wirkstoffabgabegeschwindigkeit nahezu beliebig eingestellt werden.

Die Herstellung des etofenamathaltigen Reservoirs und des darauf basierenden Pflasters kann z.B. wie folgt durchgeführt werden: Die Pflastergrundstoffe (Polymer, Harz und Schleppmittel) werden in ein geeignetes Lösegefäß eingebracht und unter Rühren in Benzin gelöst. Es resultiert eine klare bis leicht getrübte Lösung 1. Die Wirkstoff-Komponente Etofenamat wird ebenfalls in geeignetem Lösungsmittel gelöst und der Polymerlösung 1 zugesetzt.

Die so erhaltene etofenamathaltige Lösung 2 wird gleichmäßig auf silikonisiertes Papier aufgebracht und zu einem Film ausgezogen. Das beschichtete Papier mit der Pflastergrundlage wird 24 Stunden an der

# EP 0 150 426 B1

Luft getrocknet und dann 1 Stunde im Umlufttrockenschrank bei 40°C aufbewahrt.

Die Erfindung betrifft auch ein Verfahren zur Herstellung der erfindungsgemäßen medizinischen Pflaster, das dadurch gekennzeichnet ist, daß man die EPDM-Polymerkomponente, ein oder mehrere Schleppmittel und ein oder mehrere Harze in einem Lösungsmittel löst, das Etofenamat ebenfalls in einem Lösungsmittel zur Lösung bringt, diese Lösungen vereinigt und die vereinigten Lösungen gleichmäßig auf eine für den Wirkstoff im wesentlichen undurchlässige Folie (Deckschicht) aufträgt und zu einem Film auszieht, die beschichtete Folie (Deckschicht) zunächst bei Raumtemperatur und anschließend bei Temperaturen bis zu 50°C trocknet und gegebenenfalls die getrocknete Folie (Deckschicht) auf der beschichteten Seite mit einer für den Wirkstoff im wesentlichen undurchlässigen abziehbaren Schutzschicht versieht.

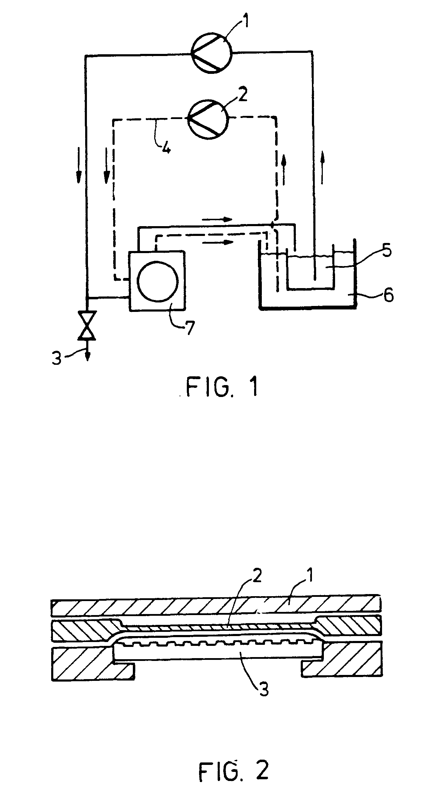

Die Bestimmungen der Wirkstoffabgaberaten erfolgten in einem Resorptionsmodell, das im folgenden und im experimentellen Teil genauer beschrieben ist. Fig. 1 zeigt ein Resorptionsmodell. Fig. 2 zeigt eine Resorptionszelle.

In Fig. 1 bedeutet 1 eine Schlauchpumpe für den Akzeptor, 2 eine Schlauchpumpe zum Temperieren, 3 die Probeentnahme, 4 den Kreislauf für die Temperierflüssigkeit, 5 das Akzeptormedium, 6 das Temperiergefäß und 7 die Resorptionszelle mit Membran.

In Fig. 2 bedeutet 1 ein undurchsichtiges Zellenmaterial, 2 eine Membrane und 3 ein Sichtfenster aus Glas, das zugleich eine Riffelplatte für as Akzeptormedium ist.

In-vitro Freisetzungsprüfung der erfindungsgemäßen Pflaster

Alle Pflaster wurden auf die gleiche Weise mit 10% Wirkstoff-Komponente hergestellt aus Polymer, Schleppmittel, Harz, gegebenenfalls Lösungsmittel (Benzin, Hexan oder Hexan/Toluol-Mischung). Die jeweils verwendeten Mengenverhältnisse sind bei den Rezepturbeschreibungen aufgeführt.

Hierzu werden alle Komponenten gelöst oder suspendiert. Als Lösungsmittel für den Wirkstoff wurden überwiegend Aceton und/oder Ethanol verwendet.

Diese Lösungen bzw. Suspensionen wurden zu dünnen Folien einer Dicke von 50—150 µm verarbeitet.

Versuchsparameter:

| | |
|---|---|
| —Akzeptormedium | Mischung aus Wasser, Ethanol, PVP, Sorbitanfettsäureester |
| —Volumen Akzeptormedium | 200 ml |
| —Temperatur Akzeptormedium | 35—36°C |
| —Pumpleistung | 16 ml/min. (Gerätekonstante) |
| —Membran | als Membran wurde die in Beispiel 3 der DE—A—3 312 735 beschriebene Folie eingesetzt |
| Resorptionsfläche | 33,18 cm² (Zellenkonstante) |

Das Akzeptormedium wurde in einem Vorratsgefäß temperiert und über Schläuche in die Resorptionszelle umgepumpt. Die Probenentnahme erfolgte zwischen der Pumpe und den Resorptionszellen. Die Probenziehung erfolgte in festgelegten Zeitabständen. Es wurden je 6 ml Probe entnommen und spektralphotometrisch vermessen. Ein Ersatz der Akzeptorflüssigkeit erfolgte nicht, da diese eine Verdünnung der Restmenge bedeuten würde.

Berechnung der Ergebnisse

Zunächst wurde eine Eichkurve von Etofenamat aufgenommen, mit deren Hilfe dann aus den für die Einzelproben gemessenen Extinktionswerten die Wirkstoffkonzentration (mg oder %) in den Einzelproben ermittelt wurde. Die Extinktionen wurden UV-spektroskopisch gemessen.

Zur Berechnung der "relativen Resorption" (Anteil "resorbierter" Wirkstoff am Gesamtgehalt des Pflasters in %) ist die Kenntnis der eingesetzten Wirkstoffmenge pro Pflasterfläche (33,18 cm²) nötig. Diese ist aus der Herstellung des Pflasters bekannt; die Menge des eingesetzten Pflasters ergibt sich aus der Differenz des Gewichts des ausgestanzten Pflasterstücks und dem Gewicht des gleich großen Stücks des silikonisierten Papiers. Aus dem Wirkstoffgehalt und dem Gewicht des eingesetzten Pflasters läßt sich die Menge Wirkstoff in 33,18 cm² Pflaster berechnen.

Aus den für die einzelnen Proben gemessenen Extinktionswerten wurde mit Hilfe einer Eichgeraden bzw. dem daraus ermittelten Faktor die Konzentration an Wirkstoff in der Probe ermittelt.

$$M_i(t) = V_t \cdot C_i + M_F(t) \, [mg]$$

$$M_F(t) = \sum_{i=0}^{i=n-1} (V_D \cdot C_i) \, [mg]$$

4

# EP 0 150 426 B1

$M_i(t)$: freigesetzte Arzneistoffmenge bis zur Zeit t[mg]
$V_t$: Volumen des Akzeptors zur Zeit t[ml]
$C_i$: Wirkstoffkonzentration in der betreffenden Probe [mg/ml]
$M_F(t)$: entnommene Wirkstoffmenge bis zur Zeit t[mg]
$V_D$: Probevolumen [ml]
n: Anzahl der Proben bis zur Zeit t
t: Versuchsdauer

Herstellungsbeschreibung

Die erfindungsgemäßen medizinischen Pflaster wurden folgendermaßen hergestellt: Das Gemisch aus Polymer, Harz und Schleppmittel wurden in einem Z-Kneter bei einer Temperatur von 120 bis 150°C vorgeknetet. Wenn die Masse eine homogene Schmelze darstellte, wurde unter Stickstoffbegasung der Wirkstoff homogen eingearbeitet. Die etofenamathaltige Schmelze wurde auf die Trägerfolie aufgetragen (Kneter).

Die erfindungsgemäßen medizinischen Pflaster wurden in einem Lösungsmittelgemisch aufgelöst, auf die Trägerfolie aufgetragen und anschließend getrocknet (Lösung).

Beispielserie A: "Standard" (Vergleich gemäß Stand der Technik DE—A—3 007 368)

Bei dieser Versuchsreihe wurde ein Styrol/Isopren/Styrol-TR-Blockcopolymerisat ("Cariflex TR 1107" von Shell Chem. Co.) als Polymer, Paraffin dünnflüssig als Schleppmittel und Polyterpenharz aus ß-Pinen als klebrigmachendes Harz eingesetzt.

Das 10% etofenamathaltige Styrol/Isopren/Styrol-TR-Blockcopolymer-Pflaster wurde in allen weiteren Versuchen als Bezugsstandard verwendet.

Die genaue Zusammensetzung der Pflastergrundlage ist in Tabelle 1 angegeben. Die Herstellung erfolgte wie vorstehend beschrieben. Die Freisetzungsraten sind in Tabelle 2 beschrieben.

### TABELLE 1
### Zusammensetzung der Standard-Formulierung

| | |
|---|---|
| Styrol/Isopren/Styrol/TR-Blockcopolymerisat | 36,0 g |
| Paraffin dünnflüssig | 45,0 g |
| Polyterpenharz aus ß-Pinen | 9,0 g |
| Etofenamat | 10,0 g |

### TABELLE 2
### Freisetzung der Standardversuchsserie in Abhängigkeit von der Zeit

| | freigesetzte Menge Etofenamat in mg/hg | | | | | | | Einwaage in Etofenamat in mg |
|---|---|---|---|---|---|---|---|---|
| | 0,5 | 1 | 1,5 | 2 | 3 | 4 | % | |
| Standard 10% | 1,44 | 2,16 | 2,70 | 3,24 | 4,63 | 4,81 | 21,20 | 22,77 |

Beispielserie B

Bei dieser Versuchsreihe wurde die Zusammensetzung der EPDM-Polymeren variiert. Die genaue Beschreibung der EPDM-Polymeren ist in Tabelle 3 angegeben.

Die Herstellung erfolgte wie vorstehend beschrieben.

Alle in Tabelle 3 aufgeführten EPDM-Polymeren wurden unter Konstanthalten des Paraffinöls bzw. Harzanteiles in der Menge nach folgendem Schema variiert:

| | A1 | A2 |
|---|---|---|
| Polymer | 36,0 g | 45,0 g |
| Paraffinöl dünnflüssig | 45,0 g | 37,5 g |
| Polyterpenharz aus ß-Pinen | 9,0 g | 7,5 g |
| Etofenamat | 10,0 g | 10,0 g |

Die Freisetzungsraten sind in Tabelle 4 beschrieben.

5

TABELLE 3

Beispielserie B: Beschreibung der eingesetzten EPDM-Polymeren.
Die beiden ersten Ziffern bezeichnen das Polymer, anstelle von×tritt die Bezeichnung der Formulierung (siehe Seite 25, A1 oder A2).

Nr.:

02×EPDM—Terpolymerisat mit ca. 45 Gew.-% Propylen, Ethylidennorbornen als Dienkomponente, Jodzahl ca. 13, Mooney Plastizität ML (1+4) 100°C:45

03×wie 02, aber mit ca. 30 Gew.-% Propylen, Jodzahl ca. 13, Mooney Plastizität ML (1+4) 100°C:35

04×EPDM—Terpolymerisat (3% Ethylidennorbornen), mit ca. 60 Gew. Teilen Propylen, Jodzahl ca. 7, Mooney Plastizität 65

05×wie 04, mit 6% Ethylidennorbornen, Jodzahl ca. 12, Mooney Plastizität 55

06×wie 04, mit 6% Dicyclopentadien, Jodzahl ca. 12, Mooney Plastizität 40

TABELLE 4

Beispielserie B: Freisetzung in Abhängigkeit von der Zeit

| Nr. | freigesetztes Etofernamat (mg) in h | | | | | | % | Etofenamat Einwaage in mg |
|---|---|---|---|---|---|---|---|---|
| | 0,5 | 1 | 1,5 | 2 | 3 | 4 | | |
| Standard | 1,44 | 2,16 | 2,70 | 3,24 | 4,63 | 1,81 | 21,20 | 22,77 |
| 01 A1 | 3,57 | 5,05 | 6,20 | 7,55 | 9,48 | 10,82 | 54,15 | 19,99 |
| 01 A2 | 2,45 | 5,71 | 7,53 | 8,83 | 11,92 | 14,00 | 45,73 | 30,62 |
| 02 A1 | 4,39 | 5,82 | 7,45 | 9,03 | 11,57 | 12,63 | 56,12 | 22,50 |
| 02 A2 | 4,79 | 6,48 | 8,44 | 10,02 | 12,85 | 14,88 | 53,58 | 27,78 |
| 03 A1 | 3,67 | 5,45 | 7,12 | 8,31 | 10,28 | 11,67 | 43,70 | 26,71 |
| 03 A2 | 3,83 | 6,05 | 8,16 | 10,96 | 12,65 | 12,65 | 47,02 | 26,90 |
| 04 A1 | 3,82 | 6,45 | 9,23 | 10,99 | 13,72 | 15,67 | 67,91 | 23,09 |
| 04 A2 | 3,72 | 6,34 | 8,41 | 10,59 | 13,46 | 15,54 | 47,86 | 32,47 |
| 05 A1 | 4,90 | 6,68 | 8,35 | 9,89 | 12,18 | 13,61 | 55,40 | 24,56 |
| 05 A2 | 3,47 | 5,69 | 8,33 | 9,71 | 12,19 | 14,10 | 47,01 | 29,99 |
| 06 A1 | 5,71 | 7,54 | 9,41 | 11,17 | 13,60 | 14,94 | 54,28 | 27,53 |
| 06 A2 | 3,93 | 6,65 | 8,52 | 10,65 | 13,35 | 15,21 | 52,70 | 28,86 |

Beispielserie C: Variation der flüssigen Komponente

Die flüssige Komponente der aus Beispielserie B ausgewählten Formulierungen wurde in der Zusammensetzung nach folgendem Schema geändert unter Beibehaltung der übrigen Rezeptur.

| | B1 | B2 |
|---|---|---|
| Polymer flüssige Komponente | wie in Beispielserie B Polybutadienöl M.G. 1500 | Polybutadienöl M.G. 1500+ Paraffinöl dünnflüssig 1:1 |
| Etofenamat | 10% | 10% |

Die genaue Zusammensetzung der Pflastergrundlagen sind in Tabelle 5, die Freisetzungsraten in Tabelle 6 angegeben.

6

# EP 0 150 426 B1

TABELLE 5
Beispielserie C: Zusammensetzung der Formulierungen

| Nr. | Polymer | fl. Komponente | | Harz | Etofenamat |
| | | fl. Polybuta-dienöl | Parafinöl dünnflüssig | | |
|---|---|---|---|---|---|
| 02A2 | 45% | — | 37,5 % | 7,5% | 10% |
| 02B1 | 45% | 37,5 % | — | 7,5% | 10% |
| 02B2 | 45% | 18,75% | 18,75% | 7,5% | 10% |
| 06A1 | 36% | — | 45% | 9% | 10% |
| 06B1 | 36% | 45% | — | 9% | 10% |
| 06B2 | 36% | 22,5 % | 22,5 % | 9% | 10% |
| 06A2 | 45% | — | 37,5 % | 7,5% | 10% |
| 06B1 | 45% | 37,5 % | — | 7,5% | 10% |
| 06B2 | 45% | 18,75% | 18,75% | 7,5% | 10% |

TABELLE 6
Beispielserie C: Freisetzung in Abhängigkeit von der Zeit

| Nr. | freigesetztes Etofenamat (mg) in h | | | | | | % | Etofenamat Einwaage in mg |
| | 0,5 | 1 | 1,5 | 2 | 3 | 4 | | |
|---|---|---|---|---|---|---|---|---|
| Standard | 1,44 | 2,16 | 2,70 | 3,24 | 4,63 | 4,81 | 21,20 | 22,77 |
| 02A2 | 4,79 | 6,48 | 8,44 | 10,02 | 12,85 | 14,88 | 53,58 | 27,78 |
| 02B1 | 3,11 | 4,69 | 6,18 | 7,57 | 10,04 | 11,95 | 42,52 | 28,10 |
| 02B2 | 3,52 | 5,15 | 7,16 | 8,60 | 11,12 | 13,24 | 42,14 | 31,42 |
| 06A1 | 5,71 | 7,54 | 9,41 | 11,17 | 13,60 | 14,94 | 54,28 | 27,53 |
| 06B1 | 2,04 | 3,68 | 5,54 | 6,80 | 9,89 | 12,28 | 35,00 | 35,08 |
| 06B2 | 1,99 | 3,47 | 5,24 | 6,59 | 9,37 | 11,46 | 40,97 | 27,96 |
| 06A2 | 3,93 | 6,65 | 8,52 | 10,65 | 13,35 | 15,21 | 52,70 | 28,86 |
| 06B1 | 2,55 | 4,73 | 6,60 | 8,31 | 11,46 | 13,62 | 44,23 | 30,80 |
| 06B2 | 2,55 | 4,58 | 6,64 | 8,22 | 11,41 | 13,88 | 42,57 | 32,59 |

Beispielserie D: Änderung des Harzanteils

TABELLE 7
Rezepturen bei geändertem Harzanteil

| Nr. | Polymer | Paraffin | Harz | Etofenamat |
|---|---|---|---|---|
| 02A2 | 45% | 37,5% | 7,5% | 10% |
| 02C1 | 43,2% | 36% | 10,8% | 10% |
| 02C2 | 41,5% | 34,6% | 13,9% | 10% |

# EP 0 150 426 B1

### TABELLE 8
### Freisetzung bei geändertem Harzanteil

| Nr. | freigesetztes Etofenamat (mg) in h | | | | | | % | Etofenamat Einwaage in mg |
|---|---|---|---|---|---|---|---|---|
| | 0,5 | 1 | 1,5 | 2 | 3 | 4 | | |
| Standard | 1,44 | 2,16 | 2,70 | 3,24 | 4,63 | 4,81 | 21,20 | 22,77 |
| 02A2 | 4,79 | 6,48 | 8,44 | 10,02 | 12,85 | 14,88 | 53,58 | 27,78 |
| 02C1 | 3,42 | 5,74 | 8,04 | 9,57 | 11,73 | 13,42 | 51,59 | 26,01 |
| 02C2 | 2,19 | 3,87 | 5,31 | 6,43 | 8,54 | 10,05 | 32,68 | 30,76 |

### Biespiel 1 (Lösung)

EPDM-Terpolymerisat, mit ca. 45 Gew.% Propylen,
Ethylidennorbornen als Dienkomponente
Mooney-Viskosität ML (1+4) 100°C:45     45,0 g
Paraffin dünnflüssig     37,5 g
Polyterpenharz aus ß-Pinen     7,5 g
Etofenamat     10,0 g
Freisetzung: nach 4 Std. 14,88 (53,58%)

### Beispiel 2 (Lösung)

EPDM-Terpolymerisat, mit ca. 30 Gew. % Propylen,
Mooney-Viskosität ML (1+4) 100°C 35     36,0 g
Paraffin dünnflüssig     45,0 g
Polyterpenharz aus ß-Pinen     9,0 g
Etofenamat     10,0 g
Freisetzung: nach 4 Std. 11,67 mg (43,7%)

### Beispiel 3 (Lösung)

EPDM-Terpolymerisat, (3% Ethylidennorbornen) mit ca.
60 Gew. Teilen Propylen Mooney
Viskosität ML (1+4) 100°C:65     36,0 g
Paraffin dünnflüssig     45,0 g
Polyterpenharz aus ß-Pinen     9,0 g
Etofenamat     10,0 g
Freisetzung: nach 4 Std. 15,67 mg (67,91%)

### Beispiel 4 (Lösung)

EPDM-Terpolymerisat (6% Ethylidennorbornen)
Mooney-Viskosität ML (1+4) 100°C:55     36,0 g
Paraffin dünnflüssig     45,0 g
Polyterpenharz aus ß-Pinen     9,0 g
Etofenamat     10,0 g
Freisetzung: nach 4 Std. 13,61 mg (55,4%)

### Beispiel 5 (Lösung)

EPDM-Terpolymerisat, mit 6% Dicyclopentadien, Mooney-
Viskosität ML (1+4) 100°C:40     36,0 g
Paraffin dünnflüssig     45,0 g
Polyterpenharz aus ß-Pinen     9,0 g
Etofenamat     10,0 g
Freisetzung: nach 4 Std. 14,94 mg (54,28%)

## Patentansprüche

1. Medizinische Pflaster zur transdermalen Verabreichung von Antiphlogistika, bestehend aus einer Deckschicht, einer abziehbaren Schutzschicht und einer den Wirkstoff, Schleppmittel und Harze enthaltenden einlagigen Reservoirschicht auf Basis von Polymeren

8

# EP 0 150 426 B1

dadurch gekennzeichnet, daß die Reservoirschicht als Polymere EPDM-Kautschuke enthält und 1 bis 30 Gew.-% des Wirkstoffs Etofenamat enthält.

2. Medizinische Pflaster nach Anspruch 1, dadurch gekennzeichnet, daß es in der Reservoirschicht 2 bis 20 Gew.-% Etofenamat enthält.

3. Medizinische Pflaster nach Anspruch 1 und 2, worin die Reservoirschicht 2 bis 15 Gew.-% Etofenamat, 30 bis 60 Gew.-% Polymerkomponente, 30 bis 60 Gew.-% Schleppmittel und 2 bis 40 Gew.-% Harze enthält, wobei sich die Komponenten zu 100 Gew.-% ergänzen.

4. Medizinische Pflaster nach Anspruch 1 bis 3, worin die EPDM-Polymere aus 20 bis 90 Gew.-Teilen Ethylen, 10 bis 80 Gew.-Teilen Propylen und 2 bis 15 Gew.-Teilen nichtkonjugiertem Dien besteht.

5. Medizinische Pflaster nach Anspruch 1 bis 4, worin das nichtkonjugierte Dien Cyclopentadien, Ethylidennorbornen oder Hexadien-1,4 ist.

6. Medizinische Pflaster nach Anspruch 1 bis 5, worin die EPDM-Polymeren ein Molekulargewicht $M_w$ von 20 000 g/mol bis $1 \times 10^6$ g/mol haben.

7. Verfahren zur Herstellung von medizinischen Pflastern nach den Ansprüchen 1 bis 6, dadurch gekennzeichnet, daß man die Polymerkomponente, bestehend aus EPDM-Polymeren, Schleppmittel und Harze in einem Lösungsmittel löst, 1 bis 30 Gew.-% Etofenamat, bezogen auf das Gemisch aus Polymeren, Schleppmittel und Harz, ebenfalls in einem Lösemittel zur Lösung bringt, diese Lösungen vereinigt und die vereinigten Lösungen gleichmäßig auf eine für Etofenamat im wesentlichen undurchlässigen Folie (Deckschicht) aufträgt und zu einem Film auszieht, die beschichtete Folie (Deckschicht) zunächst bei Raumtemperatur und anschließend bei Temperaturen bis zu 50°C trocknet und gegebenenfalls die getrocknete Folie (Deckschicht) auf der beschichteten Seite mit einer für Etofenamat im wesentlichen undurchlässigen abziehbaren Schutzschicht versieht.

## Revendications

1. Emplâtre médicinal pour l'administration transdermique d'antiphlogistiques, constitué d'une couche de revêtement, d'une couche protectrice amovible et d'une monocouche de réserve à base de polymères, contenant la substance active, des agents d'entraînement et des résines, caractérisé en ce que la couche de réserve contient comme polymères des caoutchoucs EPDM et comme substance active 1 à 30% en poids d'étofénamate.

2. Emplâtre médicinal suivant la revendication 1, caractérisé en ce que la couche de réserve contient 2 à 20% en poids d'étofénamate.

3. Emplâtre médicinal suivant les revendications 1 et 2, dans lequel la couche de réserve contient 2 à 15% en poids d'étofénamate, 30 à 60% en poids de composant polymérique, 30 à 60% en poids d'agent d'entraînement et 2 à 40% en poids de résines, les composants formant un total de 100% en poids.

4. Emplâtre médicinal suivant les revendications 1 à 3, dans lequel les polymères EPDM sont formés de 20 à 90 parties en poids d'éthylène, 10 à 80 parties en poids de propylène et 2 à 15 parties en poids de diène non conjugué.

5. Emplâtre médicinal suivant les revendications 1 à 4, dans lequel le diène non conjugué est le cyclopentadiène, l'éthylidène-norbornène ou l'hexadiène-1,4.

6. Emplâtre médicinal suivant les revendications 1 à 5, dans lequel les polymères EPDM ont un poids moléculaire $M_p$ de 20 000 g/mole à $1 \times 10^6$ g/mole.

7. Procédé de production d'emplâtres médicinaux suivant les revendications 1 à 6, caractérisé en ce qu'on dissout le composant polymérique, constitué de polymères EPDM, l'agent d'entraînement et des résines dans un solvant, on met en solution également dans un solvant 1 à 30% en poids d'étofénamate, par rapport au mélange de polymères, d'agent d'entraînement et de résine, on réunit ces solutions, et on applique uniformément les solutions réunies sur une feuille essentiellement imperméable à l'étofénamate (couche de revêtement) et on les étale en un film, on fait sécher la feuille enduite (couche de revêtement) tout d'abord à la température ambiante puis à des températures allant jusqu'à 50°C, et, le cas échéant, on munit la feuille séchée (couche de revêtement), sur le côté revêtu, d'une couche protectrice amovible principalement imperméable à l'étofénamate.

## Claims

1. Medicinal plaster for the transdermal administration of antiinflammatory agents, comprising a covering layer, a protective layer which can be pulled off and a one-layer reservoir layer based on polymers and containing the active compound, entraining agents and resins, characterized in that the reservoir layer contains EPDM rubbers as polymers and contains 1 to 30% by weight of the active compound etofenamate.

2. Medicinal plaster according to Claim 1, characterized in that it contains 2 to 20% by weight of etofenamate in the reservoir layer.

3. Medicinal plaster according to Claim 1 and 2, in which the reservoir layer contains 2 to 15% by weight of etofenamate, 30 to 60% by weight of polymer component, 30 to 60% by weight of entraining agent and 2 to 40% by weight of resins, the components adding up to 100% by weight.

4. Medicinal plaster according to Claim 1 to 3, in which the EPDM polymers comprise 20 to 90 parts by weight of ethylene, 10 to 80 parts by weight of propylene and 2 to 15 parts by weight of non-conjugated diene.

5. Medicinal plaster according to Claim 1 to 4, in which the non-conjugated diene is cyclopentadiene, ethylidenenorbornene or 1,4-hexadiene.

6. Medicinal plaster according to Claim 1 to 5, in which the EPDM polymers have a molecular weight $M_w$ of 20,000 g/mol to $1 \times 10^6$ g/mol.

7. Process for the production of medicinal plasters according to Claims 1 to 6, characterized in that the polymer component, comprising EPDM polymers, entraining agents and resins is dissolved in a solvent, 1 to 30% by weight of etofenamate, relative to the mixture of polymers, entraining agents and resin, is also brought into solution in a solvent, these solutions are combined and the combined solutions are uniformly applied to a layer (covering layer) which is essentially impermeable to etofenamate and drawn out to give a film, the coated layer (covering layer) is dried first at room temperature and then at temperatures up to 50°C and, if appropriate, the dried layer (covering layer) is provided on the coated side with a protective layer which can be pulled off and which is essentially impermeable to etofenamate.

FIG. 1

FIG. 2